**Europäisches Patentamt**

**European Patent Office**

**Office européen des brevets**

(19)

(11) **EP 1 486 190 A1**

(12) **EUROPEAN PATENT APPLICATION**
published in accordance with Art. 158(3) EPC

(43) Date of publication:
**15.12.2004 Bulletin 2004/51**

(21) Application number: **03705308.9**

(22) Date of filing: **19.02.2003**

(51) Int Cl.⁷: $A61F\ 13/53$

(86) International application number:
**PCT/JP2003/001762**

(87) International publication number:
**WO 2003/070142 (28.08.2003 Gazette 2003/35)**

(84) Designated Contracting States:
**AT BE BG CH CY CZ DE DK EE ES FI FR GB GR HU IE IT LI LU MC NL PT SE SI SK TR**

(30) Priority: **25.02.2002 JP 2002047630**

(71) Applicant: **TORAY INDUSTRIES, INC.**
**Tokyo 103-8666 (JP)**

(72) Inventors:
- **YAMAMOTO, Hideyuki**
  **Kusatsu-shi, Shiga 525-0032 (JP)**
- **SANO, Shinji**
  **Ibaraki-shi, Osaka 567-0031 (JP)**
- **KOBAYASHI, Kiyoshi**
  **Otsu-shi, Shiga 520-2145 (JP)**

- **MORI, Koji**
  **Ibaraki-shi, Osaka 567-0031 (JP)**
- **HORIIKE, Taizo**
  **Otsu-shi, Shiga 520-0863 (JP)**
- **AIYAMA, Kazunori**
  **Otsu-shi, Shiga 520-0845 (JP)**
- **MIEDA, Koichi**
  **Hirakata-shi, Osaka 573-0005 (JP)**
- **SUGINO, Tomoshige**
  **Ritto-shi, Shiga 520-3015 (JP)**

(74) Representative: **Sturt, Clifford Mark et al**
**Miller Sturt Kenyon**
**9 John Street**
**London WC1N 2ES (GB)**

(54) **BODY FLUID ABSORBING PRODUCT, AND DIAPER**

(57) A body fluid absorbing product includes a breathable top sheet and a water-absorbing component. The water-absorbing component includes a plurality of synthetic nonwoven fabric layers that have a fineness in the range of 0.01 dtex to 2 dtex. For example, the body fluid absorbing product is used as a diaper that can be reused by washing in hospitals and homes and exhibits excellent dry feeling to skin.

EP 1 486 190 A1

## Description

BACKGROUND OF THE INVENTION

1. Field of the Invention

The present invention relates to sanitary products such as a diaper composed of fiber. In particular, the present invention relates to sanitary products such as a diaper that can be repeatedly used in hospitals and homes by washing.

2. Description of the Related Art

Cotton diapers such as dobby textile diapers are repeatedly used by washing. Unfortunately, the dobby textile diapers exhibit poor water absorption. After urination, the surface of a dobby textile diaper is wet, and the wet surface makes a user uncomfortable.

In recent years, paper diapers have become widespread because of great facility in use and less discomfort. The paper diaper is composed of a top sheet of polypropylene spunbonded nonwoven fabric, or meltblown nonwoven fabric that comes into contact with skin and a water absorbent primarily composed of an acrylic polymer. Since the paper diapers cannot be reused, the used paper diapers cause social issues on industrial waste with an increase in amount used.

SUMMARY OF THE INVENTION

An object of the present invention is to provide a body fluid absorbing product and a diaper that can be reused by washing and exhibits excellent dry feeling to skin.

A body fluid absorbing product according to the present invention includes a breathable top sheet; and a water-absorbing component including a plurality of synthetic nonwoven fabric layers having a fineness in the range of 0.01 dtex to 2 dtex. Preferably, the body fluid absorbing product is a diaper.

The body fluid absorbing product (diaper) of the present invention is a combination of the water-absorbing component and the top sheet having different diffused areas of a droplet at two faces, a face in contact with skin having a smaller diffused area while a face in contact with the water-absorbing component having a larger diffused area.

The top sheet facilitates migration of urine eliminated by a user from an inner face in contact with skin to the water-absorbing component and suppresses migration of the urine from the water-absorbing component to the inner face. Furthermore, urine is rapidly diffused along the plane of the water-absorbing component and does not bleed out from the water-absorbing component by pressure.

Since this diaper significantly rapidly absorbs urine eliminated from the user and retains the urine under pressure, the face in contact with skin of the top sheet exhibits satisfactory dry feeling and makes the user comfortable.

DESCRIPTION OF THE PREFERRED EMBODIMENTS

A body fluid absorbing product according to the present invention includes a breathable top sheet and a water-absorbing component. The top sheet comes into direct contact with private parts and a backside of a human body, and thus first comes into contact with fresh feces and urine eliminated. The water-absorbing component is in contact with the top sheet and remote from skin.

The body fluid absorbing product is characterized by the water-absorbing component. The water-absorbing component preferably used in the present invention includes a plurality of synthetic nonwoven fabric layers. Natural fibers such as cotton, wool, and hemp are not used in the present invention. The water-absorbing component may be composed of semi-synthetic fibers derived from natural products, such as a rayon fiber and an acetate fiber, and pure synthetic fibers, i.e., polyester, polyamide, acrylic, polypropylene, and polyethylene fibers. Pure synthetic fibers having poor water absorbency are preferably used in the present invention. Since natural fibers, which have water absorbency, are wetted in use, a user feels discomfort. Furthermore, natural fibers are readily damaged by washing during repeated use. Also, semi-synthetic fibers have water absorbency that is less than that of the natural fibers, and are not so preferably used in the present invention.

In the present invention, nonwoven fabrics are preferable. The nonwoven fabrics can easily control the thickness and the void content of the water-absorbing component and are relatively inexpensive. The nonwoven fabric is composed of fibers and voids. In order to improve the water absorbency, the void content and the surface area of the fibers are preferably increased to hold water by the capillary phenomenon.

In the present invention, the fineness of the fiber in the water-absorbing component is important. A lower fineness causes a larger surface area of the fibers for the same weight and smaller voids between the fibers, resulting in improved water retain ability due to the capillary phenomenon. The fineness is preferably in the range of 0.01 dtex to 2 dtex. A fineness less than 0.01 dtex causes deterioration of washing ability and a drying property due to excess water absorbency, and deterioration of void formation due to insufficient rigidity of monofilaments. A fineness exceeding 2 dtex, namely, a large diameter of the fibers causes formation of large voids that exhibit poor water retain ability. Within the above range, the body fluid absorbing product or diaper according to the present invention can rapidly absorb water and does not bleed out to skin even if pressure is applied to the body fluid absorbing product. Thus, a user feels the dryness of skin and comfort.

The water-absorbing component comprises a plurality of layers. These layers may be of the same type or different types. For example, fibers of these layers have different thickness. The water absorbency required for the body fluid absorbing product can be readily adjusted by the number of the layers. The number of the layers may be partially changed to control the water absorbency at a portion of the body fluid absorbing product. In such a layer structure, a larger number of layers are placed at a position that requires higher water absorbency whereas a smaller number of layers are placed at a position that does not require higher water absorbency, in the body fluid absorbing product. The water-absorbing component having such a layer structure is generally flexible and can be readily bent because of displacement of layers during bending. The number of the layers is not limited in the present invention and preferably is in the range of 2 to 20. The water-absorbing component composed of these layers may be partially stitched up or bonded by needle punching.

The body fluid absorbing product according to the present invention is preferably used as a diaper, although the product may be used for absorption of any body fluid. The body fluid absorbing product is preferably used as any type of diaper, i.e., a geriatric diaper, an infant diaper, a child diaper, and a mature diaper.

The water-absorbing component according to the present invention preferably has a compressibility of 30% or less across the thickness. A compressibility exceeding 30% causes ready deformation by pressure with a finger when the water-absorbing component contains water. Thus, the water bleeds out from the water-absorbing component, and a user does not feel dryness. More preferably, the compressibility is 20% or less and most preferably 10% or less. If the compressibility is 0%, the body fluid absorbing product is too rigid to use. Thus, the lower limit of the compressibility is preferably 2%.

The compressibility of the water-absorbing component is measured as follows. Water-absorbing components are stacked into a thickness of 5 cm or more and preliminarily compressed under a load of 10 g/cm$^2$ to remove wrinkles and spaces. The thickness T1 of the stack under a load of 0.5 g/cm$^2$ and then the thickness T2 under a load of 10 g/cm$^2$ are measured. The compressibility is calculated from the equation:

$$\text{Compression set (\%)} = \{(T1\text{-}T2)/T1\} \times 100$$

The density of the water-absorbing component of the present invention is not limited, and is preferably in the range of 0.07 g/cm$^3$ to 0.5 g/cm$^3$. A density less than 0.07 g/cm$^3$ causes high water retainability by the capillary phenomenon and large compression deformation due to increased voids, and undesirably bleeds out water by compression. A density exceeding 0.5 g/cm$^3$ impairs water retainability and causes increases in weight, rigidity, and price due to an increased amount of fiber. The density is more preferably in the range of 0.1 g/cm$^3$ to 0.4 g/cm$^3$.

The volume of the water-absorbing component for 100 cm$^2$ is measured as follows. Water-absorbing components are stacked into a thickness of 5 cm or more and preliminarily compressed under a load of 10 g/cm$^2$ to remove wrinkles and spaces. The thickness, the length, and the width of the stack are measured under a load of 0.5 g/cm$^2$, and are divided by the number of the water-absorbing components. The water-absorbing component includes a plurality of layers in the present invention, as described above. In the measurement, these layers are regarded as a single unit and a plurality of water-absorbing components are stacked into a thickness of 5 cm or more. The volume of the fiber for 100 cm$^2$ is calculated from the observed weight and volume of the textile and the density of the fiber. If the sample does not have the area of 100 cm$^2$, the volume is calculated by conversion. The void volume for 100 cm$^2$ of the water-absorbing component is calculated from a difference between the volume for 100 cm$^2$ of the water-absorbing component and the volume for 100 cm$^2$ of the fiber.

Preferably, the water-absorbing component has a void volume of at least 10 cm$^3$ for 100 cm$^2$. The void volume represents the difference between the volume of the textile and the volume of the fiber and indicates water absorbency by the capillary phenomenon. A larger void volume is preferable in the present invention.

According to an empirical rule, a void volume less than 10 cm$^3$ for 100 cm$^2$ does not achieve satisfactory water absorption. In such a case, during the use of the body fluid absorbing product as a diaper, liquid readily bleeds out from the diaper. Thus, the body fluid absorbing product is not suitable for a diaper. A human eliminates 150 ml to 200 ml of urine every time, while a typical diaper contains 300 cm$^2$ to 1,000 cm$^2$ of water-absorbing component. Thus, a maximum void volume of 500 cm$^3$ for 100 cm$^2$ can absorb urine corresponding to double elimination volumes. The upper limit of the void volume is not set in the present invention. However, a larger void volume will cause disappearance of capillary phenomenon and large compression by pressure due to formation of large voids, and thus water will readily bleed out by compression.

The water-absorbing component is preferably thinner in view of usability but is preferably thicker in view of the void volume. The minimum thickness must be 1 mm and the maximum thickness is preferably 20 mm. A minimum thickness less than 1 mm does not ensure a void volume that is necessary for satisfactory water absorption. A maximum thickness exceeding 20 mm is not preferable in view of usability, feeling during use, and price, regardless of a large void volume sufficient for satisfactory water absorption. The thickness of the water-absorbing component is more preferably in the range of 3 mm to 15 mm.

In the water-absorbing component according to the present invention, preferably, a hydrophilic agent is coated on the surfaces of fibers constituting the water-absorbing component, and the water absorbency of the water-absorbing component by a Byreck method (JIS-L-1096 Method A) is at least 50 mm. Any hydrophilic agent may be used in the present invention and preferably has high durability to washing. Examples of hydrophilic agents include silicone, polyester, polyamide, acrylic, and urethane hydrophilic agents; cationic, anionic, and nonionic hydrophilic agents; and monomeric, oligomeric, and polymeric hydrophilic agents. For example, a nonionic hydrophilic agent RANOGEN KRN-6 or a polyester hydrophilic agent TO-SR-1 (these are commercial names and are made by Takamatsu Oil & Fat Co., Ltd.) is coated on the surface of the fiber. A water absorbency less than 50 mm by the Byreck method causes poor water diffusion and absorption, resulting in water leakage from the water-absorbing component. Preferably, the water absorbency is at least 200 mm.

Preferably, the water-absorbing component has a water absorption rate of at least 100% and more preferably at least 250% on the weight basis to ensure satisfactory water absorption. The water absorption rate is measured according to JIS-L-1912 as follows. The dry weight of the water-absorbing component is measured and then the water-absorbing component is immersed in water for 5 minutes. The water-absorbing component is removed from the water and is dried for 20 minutes indoors. The weight of the water-absorbing component is then measured. The water absorption rate is the percentage of the absorbed water to the dry weight of the water-absorbing component. For example, when the water-absorbing component with a dry weight of 100 g absorbs 100 g of water (total weight of the water-absorbing component: 200 g), the water absorption rate is 100%.

In the present invention, the total areal weight of the fabric layers of the water-absorbing component is preferably at least 500 g/m$^2$. The total areal weight is the sum of the masses per unit area (g/m$^2$) of all the layers. A part composed of a single layer may have an areal weight of less than 500 g/m$^2$. A part composed of a plurality of layers preferably has a large total areal weight to absorb a large amount of water. A total areal weight less than 500 g/m$^2$ at the plurality of layers causes insufficient water absorption. A total areal weight exceeding 2,000 g/m$^2$ causes a significantly large thickness of the water-absorbing component that impairs fitting to human bodies.

The fiber used in the water-absorbing component may have any cross-sectional shape. For example, the cross-sectional shape may be solid, hollow, T, flat, tear, or multilobal, i.e., trilobal, tetralobal, hexalobal, or octalobal. Cross-sectional shapes that cause a large surface area of the fiber are more preferable, for example, T, H, $\pi$, tear, and multilobal (tetralobal, hexalobal, and octalobal), in order to retain a large amount of water.

The water-absorbing component is composed of a plurality of nonwoven fabric layers. The nonwoven fabric may be prepared by any bonding method of fibers, for example, needle punching, water-jet punching, thermal fixation, and adhesive bonding. A thin fiber with a fineness of 1 dtex or less may be prepared by melt blowing, spun bonding, chemical extraction of the matrix portion of a matrix fiber, or disintegration of a compound fiber by a high-pressure water stream.

Preferably, the nonwoven fabric is prepared by disintegration of short fiber webs of a separable compound fiber composed of two or more polymers that are not compatible with each other by a high-pressure water stream. This process simultaneously achieves the formation of a stable nonwoven fabric structure and disintegration of the compound fiber with reduced production cost. The use of thin fibers in the preparation of the nonwoven fabric is not preferable since the thin fibers causes problems when they pass through a carding machine.

A melt blow long-fiber nonwoven fabric has a desirable fineness. The melt blow process, however, requires a complicated process for polyester and nylon that are preferable materials as water-absorbing components, and the resulting nonwoven fabric is not so durable during washing. From a matrix compound fiber, fine fibers can be prepared by matrix dissolution. However, this process is complicated and results in increased process cost. Thus, in the present invention, disintegration of the separable compound fiber by a high-pressure water stream is preferable. It is preferable that the separable compound fibers be disintegrated and entangled at a lower hydraulic pressure. However, at least 5 MPa is required. A fiber that can be disintegrated at a pressure less than 5 MPa causes disintegration during a stretching step of a raw fiber; hence, such a fiber cannot be produced. In the present invention, the maximum hydraulic pressure is about 25 MPa. A hydraulic pressure exceeding the maximum causes a decrease in void volume due to dense packing.

The separable compound fiber composed of two incompatible polymers preferably used in the present invention will now be described. The two incompatible polymers may have any combination that can be separated by external force such as a water stream. Examples of combinations include polyester-nylon, polyester-polyolefin. and polyester-polystyrene. These polymers are generally used in separable compound fibers.

Examples of acid components of polyester polymers include aromatic dicarboxylic acids, i.e., telephthalic acid, isophthalic acid, 2.6-naphthalene dicarboxylic acid, and esters thereof; and aliphatic carboxylic acids, i.e., adipic acid, cebacic acid, and esters thereof. Examples of glycol components of the polyester polymers include ethylene glycol, diethylene glycol, triethylene glycol, 1,4-butanediol, neopentyl glycol, and 1,4-cyclohexane-dimethanol. The polyester polymers may be a homopolyester or copolyester. The copolyester may contain paraoxybenzoic acid, 5-sodium sulfoisophthalic acid, polyalkylglycol, pentaerythritol, and Bisphenol A.

Examples of nylon polymers include nylon-4, nylon-46, nylon-6, nylon-66, nylon-610, nylon-11, nylon-12, poly(metaxylene adipamide) (MXD-6), poly(paraxylene decanamide) (PXD12), poly(biscyclohexylmethane decanamide) (PCM-12), and copolyamides having structural units composed of these monomers.

Examples of olefin polymers include polyethylene, polypropylene, and polybutylene.

Among these polymers, a polyethylene terephthalate copolymer containing 5-sodiumsulfoisophthalic acid, polypropylene terephthalate, and polybutylene terephthalate are preferable as polyester polymers, and nylon-6 is preferable as a nylon polymer. A separable compound fiber of a polyester polymer and a nylon polymer has adequate contact at interfaces between these polymers. This separable compound fiber (short fiber) is not disintegrated in a carding machine but is disintegrated by water jet punching. Another preferable combination in the separable compound fiber is a polyester polymer and polypropylene or polyethylene. The compound fiber may have any shape without restriction.

These polymers may contain other additives, such as a delustrant, pigment, flameproofing agent, deodorant, antistatic agent, antioxidant, ultraviolet absorber, and hydrophilic agent within the scope of the present invention.

The fiber in the water-absorbing component preferably is antibacterial. For example, the fiber preferably contains an organic or inorganic antibacterial agent that prevents propagation of, for example, Escherichia coli and Staphylococcus aureus. Among antibacterial agents, organic agents such as quaternary ammonium salts and chlorhexidine and inorganic agents such as silver zeolite and copper sulfide are preferable in consideration of safety. The antibacterial agent also prevents offensive odor caused by propagation of bacteria. The antibacterial agent is may be incorporated in the fiber by compounding into the fiber or coating with a binder onto the fiber in a postprocess. The antibacterial activity is measured by a method determined by Japanese Association for the Function Evaluation of Textiles. Preferably, the bacteriostatic activity value is at least 2.2.

The textile water-absorbing component according to the present invention preferably has a longitudinal or lateral shrinkage factor of 6% or less after 10 industrial washing operations. A shrinkage factor exceeding 6% causes noticeable deformation of the water-absorbing component that is not suitable for repeated use. In a diaper including a water-absorbing component having such a high shrinkage factor, puckering occurs due to a difference in shrinkage from the top sheet and other textile materials that are stitched. The puckering impairs its commercial value and causes a back flow of water.

The industrial washing in the present invention represents the following cycle washing test that includes washing at 60°C for 75 minutes, dewatering for 2 minutes, first rinsing at 50°C for 15 minutes, dewatering for 2 minutes, second rinsing at 35°C for 15 minutes, dewatering for 2 minutes, third rinsing at room temperature for 15 minutes, and dewatering for 2 minutes. This cycle corresponds to five times of industrial washing. Thus, two cycles corresponds to ten times of industrial washing.

In order to obtain a stable water-absorbing component that is not deformed during such a severe washing test, the textile water-absorbing component must be preliminarily shrank by thermal treatment at a temperature that is higher than temperatures during the industrial washing. Specifically, after a textile is cut into a predetermined shape or after a textile is shaped into a product, it is preliminarily shrank by dry hot air, wet hot air, or hot water at a temperature higher than the thermal history during the industrial washing. In general, washing is performed in hot water. Thus, the heat treatment is preferably performed in hot water at 80°C or more. If the thermal treatment is performed at a higher temperature, the treating time may be shorter than the washing time; however, the treating time equal to or longer than the washing time is more effective.

The textile water-absorbing component may be treated by any process, for example, a continuous process or a batch process. The continuous thermal process may be performed in a restricted process that restricts the lateral shrinkage within a predetermined range or in a nonrestricted process that does not restrict the lateral shrinkage. The thermal treatment is preferably performed without tension to reduce the shrinkage factor as much as possible. More preferably, the thermal treatment is performed without longitudinal tension and lateral tension to achieve a shrinkage factor of 6% or less in the longitudinal and lateral directions. Examples of heating apparatuses used for the thermal treatment are a liquid-flow dyeing machine for hot water treatment and a sanforizing machine for dry hot air treatment. A hot water washer and a tumbler dry heating machine that can treat the textile without tension are more preferable.

In the present invention, the body fluid absorbing product preferably further includes a water-draining layer composed of nonwoven fabric having a large fineness. Since the nonwoven fabric of the water-draining layer is composed of thicker fibers compared with the water-absorbing component, the water-draining layer has voids larger than those in the water-absorbing component. In a stack of the water-absorbing component and the water-draining layer, water absorbed in the diaper is retained in the water-absorbing component exhibiting a more noticeable capillary phenomenon in an ordinary state, but bleeds out from the water-absorbing component by the action of a large compressive force. The water-draining layer absorbs such bleeding water before the bleeding water reaches skin. Thus, the dry feeling of skin is maintained. The water-draining layer composed of nonwoven fabric preferably has a fineness in the range of 5 dtex to 100 dtex. A fineness less than 5 dtex does not cause a large difference in void size between the water-draining layer and the water-absorbing component. Thus, water cannot be retained only in the water-absorbing component in a normal state. A fineness exceeding 100 dtex requires a special machine for preparation of the nonwoven fabric. More preferably, the fineness is in the range of 5 dtex to 20 dtex.

It is not preferable that the water-draining layer lies at an inner face in contact with skin. It is preferable that water bleeding out from the water-absorbing component bleeds away from the skin, not toward the skin. Thus, the water-draining layer preferably lies at a side remote from the skin, in other words, remote from the top sheet.

Alternatively, the water-draining layer preferably lies as an interlayer of the water-absorbing component. In this structure, water bleeding out from the water-absorbing component is absorbed in the internal water-draining layer. Thus, bleeding of water from the inner face and from the other face remote from the skin can be reduced.

One of the characteristic features in the present invention is the use of a significantly suitable textile in the top sheet. This textile facilitates migration of liquid from the inner face (not in contact with the water-absorbing component) to the water-absorbing component but inhibits migration from the water-absorbing component to the inner face. More specifically, the top sheet is composed of a limited textile: When 0.1 ml of droplet is gently placed onto an inner face that comes into contact with skin, the diffused area of the droplet at an outer face of the water-absorbing component is at least two times the diffused area at the inner face. This means that the diffusion area is larger at the outer face of the water-absorbing component than the inner face. As a result, the textile facilitates migration of liquid from the inner face to the water-absorbing component but inhibits migration from the water-absorbing component to the inner face. Since, this phenomenon is accelerated by a larger ratio of the diffused areas, the diffused area at the water-absorbing component is more preferably at least four times the diffused area at a face in contact with skin.

The diffused area is measured as follows. Color water (0.1 ml), for example, containing red ink is gently placed from a height of 5 mm or less to the inner face with an injection syringe or a dropping pipette. The color water is diffused at the inner face that comes into contact with skin, and simultaneously migrates toward the reverse side, namely, the water-absorbing component side, and is diffused at the surface of the water-absorbing component. One minute after placing the color water, the area of the red water is measured at both the inner face and the face of water-absorbing component side.

In the present invention, it is preferable to facilitate the migration of the liquid from the skin to the water-absorbing component and to suppress the migration of the liquid from the water-absorbing component to the inner face. In addition, it is preferable that the liquid moves to the water-absorbing component as much as rapid. When 0.1 ml of droplet is gently placed onto the inner face, the droplet is absorbed into the textile preferably within 5 seconds, more preferably within 4 seconds, and most preferably within 1 second. The time when the droplet is absorbed into the textile is measured as follows. Color water (0.1 ml) containing color ink is gently placed from a height of 5 mm or less to the inner face with an injection syringe or a dropping pipette. The droplet first remains on the top sheet and then disappears by being absorbed into the textile. The time when the droplet disappears is measured.

The textile structure of the top sheet preferably exhibits the capillary phenomenon as follows. A first textile structure of the top sheet has a plurality of layers. A layer in contact with the water-absorbing component is denser than a layer that comes into contact with the skin (away from the water-absorbing component). This structure is achieved by, for example, a double layer knit textile in which the inner layer (at the skin side) has a mesh structure whereas the outer layer at the water-absorbing component has a flat structure.

A second textile structure of the top sheet has an irregular surface at a face in contact with skin, the difference between the peak and the bottom of the irregular surface being at least 200 μm. When the irregularity at a face in contact with skin is more noticeable than that at the water-absorbing component side, the liquid moves from the more irregular surface to the less irregular surface by the capillary phenomenon. This structure is achieved by, for example, a double layer knit textile in which the inner layer at the skin side has a mesh structure whereas the outer layer at the water-absorbing component has a flat structure. Alternatively, the structure is achieved by a textile with two pile fabric surfaces, the pile at the inner face being longer than that at the water-absorbing component side. The pile at the water-absorbing component side is not always necessary. The difference between the top and the bottom in the irregular surface is determined by microscopy of a cross-section of the textile, the thickest position being the top whereas the thinnest position being the bottom.

A third textile structure of the top sheet has a variable fineness that varies across the thickness and is smaller at a face in contact with the water-absorbing component. Preferably, the structure has a fineness profile across the thickness, more specifically; the fineness decreases from the inner face toward the water-absorbing component. More preferably, the fineness of a fiber, in contact with the water-absorbing component, of the top sheet is in the range of 0.01 dtex and 5.0 dtex, and the fineness of a fiber not in contact with the water-absorbing component is at least 1.2 times the fineness of the fiber in contact with the water-absorbing component. More preferably, the fineness at the water-absorbing component is in the range of 0.1 dtex to 2.0 dtex.

The textile structure may contain the first to third structures alone or in combination. Fibers suitable for these structures are synthetic fibers such as polyester, nylon, acrylic, polypropylene, and polyethylene not having water absorbency in order to achieve a high degree of dry feeling of the skin, although natural fibers such as cotton, wool, and hemp may also be used. Polyester fiber is more preferable.

The textile according to the present invention is preferably coated with a hydrophilic agent. The hydrophilic agent facilitates absorption of water or urine into the top sheet and migration of the water to the water-absorbing component. Any hydrophilic agent may be used in the present invention and preferably has high durability to washing. Examples of hydrophilic agents include silicone, polyester, polyamide, acrylic, and urethane hydrophilic agents; cationic, anionic, and nonionic hydrophilic agents; and monomeric, oligomeric, and polymeric hydrophilic agents. For example, a nonionic hydrophilic agent RANOGEN KRN-6 or a polyester hydrophilic agent TO-SR-1 (these are commercial names and are made by Takamatsu Oil & Fat Co., Ltd.) is coated on the surface of the fiber.

Preferably, the top sheet of the present invention is water-repellent. Although the water repellency of the top sheet retards water absorption, it suppresses back flow of water retained in the water-absorbing component to skin. The water repellency may be imparted to the top sheet by the use of water-repellent fibers such as polyester, polypropylene, and polyethylene or by coating a fluorinated, silicone, or polyolefinic water repellent onto the fiber surfaces.

Preferably, the top sheet has an air permeability of at least 300 $cm^3/(cm^2 \cdot sec)$, wherein the air permeability is measured according to JIS L 1096 A. A low air permeability may preclude water permeation or may cause surface deterioration of a user. A high air permeability facilitates migration of urine to the water-absorbing component. More preferably, the top sheet has an air permeability of at least 500 $cm^3/(cm^2 \cdot sec)$. An excess air permeability causes contact of the wet textile layer with user's skin that will make the user uncomfortable. Thus, the upper limit of the air permeability is preferably about 5,000 $cm^3/(cm^2 \cdot sec)$.

Since the diaper according to the present invention is repeatedly washed for reuse, the water absorption rate of the top sheet is preferably 5 seconds or less after 10 industrial washing operations. In order to maintain this water absorption rate, hydrophilic agents durable for washing must be used. Such hydrophilic agents are described above.

A water-impermeable back sheet is preferably used in the present invention. In such a case, the water-absorbing component is disposed between the top sheet and the back sheet. The back sheet generally comes into contact with a diaper cover or underwear and prevents leakage of eliminated feces and urine to the diaper cover or the underwear. Examples of the back sheets include a textile of a woven or knitted substrate coated with urethane or rubber, and water-impermeable air-permeable textile of a woven or knitted substrate covered with a microporous urethane or polytetrafluoroethylene (PTFE) film.

The back sheet must have liquid impermeability for preventing leakage of urine from the water-absorbing component. The back sheet may be a woven fabric, a knit fabric, a nonwoven fabric, a film, or a laminate thereof. Since the single use of the woven fabric, the knit fabric, or the nonwoven fabric does not show satisfactory liquid impermeability, the fabric is covered with a rubber, polyolefin, fluorinated, or silicone film or coated with a rubber, polyolefin, fluorinated, silicone, or foamed resin. Furthermore, such a layer structure exhibits water repellency.

Preferably, the back sheet has a water vapor permeability of at least 4,000 g/($m^2 \cdot 24$ hours) to prevent sweaty feeling caused by urine. The back sheet preferably has durability to the sweaty. Thus, the back sheet preferably has a limit hydraulic pressure of at least 5 kPa according to JIS-L-1092. Such a back sheet is prepared by coating porous polyurethane such as "ENTRANT" (by Toray Industries, Inc.) onto a woven, knitted, or nonwoven fabric substrate, or by laminating a porous fluorinated film such as "GORE-TEX" (by W.L. Gore & Associates) with the substrate.

Preferably, the top sheet, the water-absorbing component, and the back sheet are partially bonded, for example, at the entire or partial edges by sewing, or at part of the inside by quilting. The sewing thread used in the sewing is preferably subjected to water repellent finishing with a paraffinic, silicone, or fluorinated water repellent to prevent wet feeling due to water absorption.

The body fluid absorbing product or diaper according to the present invention preferably has a structure preventing side leakage, in order to reduce leakage of urine that is eliminated at a rate higher than the absorption rate of the water-absorbing component of the diaper, from a gap between the diaper and the skin of a user reclining on a bed. Specifically, a stretchy member of rubber or the like is provided around the edges of the diaper to ensure close contact between the diaper and the skin.

In the present invention, preferably, the back sheet is provided with an antiskid member at the outer face. The antiskid member causes proper friction to a textile such as a nightwear to prevent slippage.

EXAMPLES

The present invention will now be described in further detail by EXAMPLES.

EXAMPLE 1

A nonwoven fabric as a water-absorbing component was prepared as follows. A compound fiber PA-31 made by Toray Industries, Inc. was prepared. The compound fiber had a cross section, like a sliced orange, and was composed of a hexalobal core of polybutylene terephthalate and six fiber segments of nylon-6 disposed between the lobes of the hexalobal core. The compound fiber before disintegration had a fineness of 1.8 dtex and an average length of 38 mm. The compound fiber was disintegrated in a carding machine to form sheets. These sheets were stacked and the stack was prepunched with a water jet puncher at a hydraulic pressure of 2 MPa one time from the front face and one time from the rear face, and then punched at a hydraulic pressure of 10 MPa two times from the front face and two times from the rear face to form a nonwoven fabric. The nonwoven fabric had an areal weight of 200 g/cm$^2$. According to scanning electron microscopy of a cross section of the nonwoven fabric, the compound fiber was partly disintegrated at interfaces between polybutylene terephthalate and nylon-6. At a disintegrated portion, the fineness of polybutylene terephthalate was 0.57 dtex, whereas the fineness of nylon-6 was 0.2 dtex. The nonwoven fabric was treated with hydrophilic agents RANOGEN KRN-6 and TO-SR-1 (commercial names; made by Takamatsu Oil & Fat Co., Ltd.) such that the fixed amount on the dry nonwoven fabric of the hydrophilic agent was 2%. The nonwoven fabric had a compressibility of 7.5% across the thickness, a density of 0.19 g/cm$^3$, a water absorbency by the Byreck method of 148 mm, and a water absorption rate of 298%. The nonwoven fabric was packed into a net and was subjected to washing treatment in hot water at 80°C for 20 minutes to preliminarily shrink. The shrunk nonwoven fabric was marked at positions with a longitudinal and lateral distance of 20 cm and was subjected to ten times of industrial washing. The longitudinal and lateral shrinkage factors measured from the distances between the marks were 4.8% and 5.4%, respectively. A plurality of the nonwoven fabrics were stacked into a total areal weight of 800 g/cm$^2$ and the stack was cut into a width of 25 cm and a length of 60 cm to prepare a water-absorbing component of a diaper according to the present invention. The water-absorbing component had a void volume of 35 cm$^3$ for 100 cm$^2$.

A water-draining layer was prepared. With a raw fiber T70 made by Toray Industries, Inc. (14.4 dtex), a nonwoven fabric with a thickness 2 mm was formed using a carding machine and a needle puncher. The nonwoven fabric was cut into a width of 25 cm and a length of 60 cm to form a water-draining layer.

As a top sheet, a "Field Sensor" (product number 1218S made by Toray Industries, Inc.) was prepared. This was composed of 36 polyethylene terephthalate filaments (84 dtex by the gross, 2.3 dtex per filament) at the inner face (not in contact with the water-absorbing component) and 72 polyethylene terephthalate filaments (110 dtex by gross, 1.5 dtex per filament) at the outer face in contact with a water-absorbing component. These filaments were knitted with a double circular knitting machine so that the outer face was flat and the inner face was a mesh. The knit was treated with a polyester hydrophilic agent to form a top sheet. On the inner face in contact with skin of the top sheet, 0.1 ml of droplet was gently placed and the diffused area was measured one minute later. The diffused area of the droplet at the outer face in contact with the water-absorbing component was 5.6 times the diffused area at the inner face in contact with skin.

A waterproof polyurethane sheet bonded to a polyester Jersey stitch cloth was prepared as a back sheet. The back sheet had a limit hydraulic pressure of 4.1 kPa and a water vapor permeability of 0 g/(m$^2$·24 hours).

The top sheet and the back sheet were cut into a double guard shape. The neck of the double guard had a width of 30 cm and a length of 60 cm. The water-absorbing component was placed between the top sheet and the back sheet, and the stack was partially stitched with a polyester multifilament thread (count of yarn: 20) with 0.5% of a fluorinated water repellent along the sides of the water-draining layer and the water-absorbing component. Furthermore, the top sheet and the back sheet were stitched with the same thread along the four sides to complete a diaper of the present invention.

The diaper was used for the following bleeding test. The diaper was horizontally placed so that the top sheet was at the upside, and 100 ml of red water containing red ink was poured on the top sheet of the diaper. Thirty seconds later, a cotton woven fabric (catechin No. 4) was placed on the top sheet and a 300-g weight was placed over an area of 10 cm by 10 cm of the cotton woven fabric. Five seconds later, the weight and the cotton woven fabric were removed. The transfer of the red ink to the cotton woven fabric was visually observed as a measure of bleeding of the liquid toward the skin. Transfer of the red ink to the cotton woven fabric was indistinctive, and the diaper exhibited satisfactory dry feeling.

EXAMPLE 2

The water-absorbing component, the water-draining layer, the top sheet, the back sheet, and the thread were prepared as in EXAMPLE 1, except that the layer structure was changed. Two water-absorbing components (400 g/m$^2$), a water-draining layer, and two water-absorbing components (400 g/m$^2$) were stacked. A diaper was prepared as in EXAMPLE 1.

The diaper was used for the bleeding test as in EXAMPLE 1. Transfer of the red ink to the cotton woven fabric was indistinctive, and the diaper exhibited satisfactory dry feeling.

EXAMPLE 3

A water-proof microporous polyurethane sheet bonded to a polyester Jersey stitch cloth was prepared as a back sheet. The back sheet had a limit hydraulic pressure of 4.1 kPa and a water vapor permeability of 0 g/(m$^2$·24 hours). A diaper was prepared as in EXAMPLE 1.

The diaper was used for the bleeding test as in EXAMPLE 1. Transfer of the red ink to the cotton woven fabric was indistinctive, and the diaper exhibited satisfactory dry feeling.

EXAMPLE 4

A diaper was prepared as in EXAMPLE 1, except that no water-draining layer was provided. The diaper was used for the bleeding test as in EXAMPLE 1. Transfer of the red ink to the cotton woven fabric was slightly observed, and the diaper exhibited satisfactory dry feeling.

EXAMPLE 5

A nonwoven fabric as a water-absorbing component was prepared as follows. A compound fiber PA-31 made by Toray Industries, Inc. (the same as that in EXAMPLE 1) was prepared. The compound fiber was disintegrated in a carding machine to form sheets. These sheets were stacked and the stack was directly punched with a water jet puncher at a hydraulic pressure of 5 MPa one time from the front face and one time from the rear face to form a nonwoven fabric. The nonwoven fabric had an areal weight of 200 g/cm$^2$, but was not tight. According to scanning electro microscopy of a cross section of the nonwoven fabric, the compound fiber was partly disintegrated at interfaces between the polybutylene terephthalate and nylon-6. The nonwoven fabric was treated with hydrophilic agents RANOGEN KRN-6 and TO-SR-1 (commercial names; made by Takamatsu Oil & Fat Co., Ltd.) such that the fixed amount of the hydrophilic agent on the dry nonwoven fabric was 2%. The nonwoven fabric had a compressibility of 35.2% across the thickness, a density of 0.05 g/cm$^3$, a water absorbency by the Byreck method of 78 mm, and a water absorption rate of 423%. The nonwoven fabric was packed into a net and was subjected to washing treatment in hot water at 80°C for 20 minutes to preliminarily shrink. A plurality of the nonwoven fabrics were stacked into a total areal weight of 800 g/cm$^2$ and the stack was cut into a width of 25 cm and a length of 60 cm to prepare a water-absorbing component of a diaper according to the present invention. A diaper was prepared as in EXAMPLE 1 except that no water-draining layer was provided. The diaper was used for the bleeding test as in EXAMPLE 1. Transfer of the red ink to the cotton woven fabric was noticeable compared with EXAMPLES 1 and 4, but the diaper exhibited satisfactory dry feeling.

COMPARATIVE EXAMPLE 1

As a water-absorbing component, a cotton dobby cloth, which is generally used as a cloth diaper, was prepared. A plurality of cotton dobby clothes were stacked into a total areal weight of 800 g/cm$^2$ and the stack was cut into a width of 25 cm and a length of 60 cm to prepare a water-absorbing component of a diaper.

A polyester taffeta (plain cloth) of 36 filaments (84 dtex by the gross, 2.3 dtex for monofilament) with an areal weight of 50 g/m$^2$ made by Toray Industries, Inc. was subjected to hydrophilic treatment to form a top sheet. On an inner face that comes into contact with skin of the top sheet, 0.1 ml of droplet was gently placed and the diffused area was measured one minute after. The diffused area of the droplet at a face in contact with the water-absorbing component was 1.0 time the diffused area at a face in contact with skin.

A diaper was prepared using the top sheet and the water-absorbing component as in EXAMPLE 1. The diaper was used for the bleeding test as in EXAMPLE 1. Transfer of the red ink to the cotton woven fabric was significantly noticeable, and the diaper did not exhibit dry feeling.

COMPARATIVE EXAMPLE 2

As a water-absorbing component, a cotton dobby cloth, which is generally used as a cloth diaper, was prepared. A plurality of cotton dobby clothes were stacked into a total areal weight of 800 g/cm$^2$ and the stack was cut into a width of 25 cm and a length of 60 cm to prepare a water-absorbing component of a diaper. A diaper was prepared using this water-absorbing component as in EXAMPLE 1. The diaper was used for the bleeding test as in EXAMPLE 1. Transfer of the red ink to the cotton woven fabric was noticeable, and the diaper did not exhibit satisfactory dry feeling.

COMPARATIVE EXAMPLE 3

The water-absorbing component, the water-draining layer, the back sheet, and the thread were prepared as in EXAMPLE 1, and a water-absorbing nonwoven fabric was prepared in place of the top sheet. The water-absorbing nonwoven fabric and the back sheet were cut into a double guard shape. A diaper was prepared as in EXAMPLE 1. The diaper was used for the bleeding test as in EXAMPLE 1. Transfer of the red ink to the cotton woven fabric was noticeable, and the diaper did not exhibit satisfactory dry feeling.

COMPARATIVE EXAMPLE 4

A water-absorbing component was prepared as follows. A needle punch nonwoven fabric with a thickness of 1 mm was prepared using a raw thread of T70-14.4 dtex made by Toray Industries, Inc. The nonwoven fabric was cut into a width of 25 cm and a length of 60 cm. A diaper was prepared using the cut nonwoven fabric as in EXAMPLE 1. The diaper was used for the bleeding test as in EXAMPLE 1. Transfer of the red ink to the cotton woven fabric was noticeable, and the diaper did not exhibit satisfactory dry feeling.

**Claims**

1. A body fluid absorbing product comprising:

   a breathable top sheet; and
   a water-absorbing component comprising a plurality of synthetic nonwoven fabric layers having a fineness in the range of 0.01 dtex to 2 dtex.

2. The body fluid absorbing product according to claim 1, being a diaper.

3. The body fluid absorbing product according to claim 1, wherein the water-absorbing component has a compressibility of 30% or less across the thickness.

4. The body fluid absorbing product according to claim 3, wherein the nonwoven fabric has a compressibility of 20% or less across the thickness.

5. The body fluid absorbing product according to claim 1, wherein the synthetic nonwoven fabric layers comprises a nonwoven fabric having a density in the range of 0.07 g/cm$^3$ to 0.5 g/cm$^3$.

6. The body fluid absorbing product according to claim 1, wherein the water-absorbing component has a void volume of at least 10 cm$^3$ for 100 cm$^2$ and has a thickness in the range of 1 mm to 20 mm.

7. The body fluid absorbing product according to claim 1, wherein a hydrophilic agent is coated on the surfaces of fibers constituting the water-absorbing component, and the water absorbency of the water-absorbing component by a Byreck method is at least 50 mm.

8. The body fluid absorbing product according to claim 7, wherein the water absorbency of the water-absorbing component by a Byreck method is at least 200 mm.

9. The body fluid absorbing product according to claim 1, wherein the water-absorbing component has a water absorption rate of at least 100% on a weight basis.

10. The body fluid absorbing product according to claim 1, wherein the total areal weight of the plurality of synthetic nonwoven fabric layers is at least 500 g/m$^2$.

11. The body fluid absorbing product according to claim 1, wherein the synthetic nonwoven fabric layers are composed of a compound fiber, elements of the compound fiber being disintegrated by water jet treatment.

12. The body fluid absorbing product according to claim 1, wherein the synthetic nonwoven fabric layers are antibacterial.

13. The body fluid absorbing product according to claim 1, wherein the water-absorbing component has a longitudinal or lateral shrinkage factor of 6% or less after 10 industrial washing operations.

14. The body fluid absorbing product according to claim 1, wherein the water-absorbing component is subjected to shrinkage heat treatment in hot water at 80°C or more.

15. The body fluid absorbing product according to claim 1, further comprising a water-draining layer composed of a nonwoven fabric having a fineness in the range of 5 dtex to 100 dtex and disposed at a side remote from the top sheet.

16. The body fluid absorbing product according to claim 1, further comprising a water-draining layer composed of nonwoven fabric having a fineness in the range of 5 dtex to 100 dtex and disposed between the plurality of synthetic nonwoven fabric layers.

17. The body fluid absorbing product according to claim 1, wherein the top sheet comprises a synthetic fiber, and when 0.1 ml of droplet is gently placed onto an inner face that comes into contact with skin, the diffused area of the droplet at an outer face in contact with the water-absorbing component is at least two times the diffused area at the inner face.

18. The body fluid absorbing product according to claim 1, wherein the body fluid absorbing product has a water absorption rate of 5 seconds or less, the water absorption rate being a time when 0.1 ml of a droplet placed on the top sheet is absorbed into the body fluid absorbing product.

19. The body fluid absorbing product according to claim 1, wherein the top sheet is a textile structure with a plurality of layers, and a layer in contact with the water-absorbing component is denser than a layer away from the water-absorbing component.

**20.** The body fluid absorbing product according to claim 1, wherein the top sheet comprises a fiber textile having an irregular surface at an inner face that comes into contact with skin, the difference between the peak and the bottom of the irregular surface being at least 200 μm.

**21.** The body fluid absorbing product according to claim 1, wherein the fineness of the top sheet varies across the thickness and is smaller at a face in contact with the water-absorbing component than a face away from the water-absorbing component.

**22.** The body fluid absorbing product according to claim 1, wherein the fineness of a fiber, in contact with the water-absorbing component, of the top sheet is in the range of 0.01 dtex and 5 dtex, and the fineness of a fiber not in contact with the water-absorbing component is at least 1.2 times the fineness of the fiber in contact with the water-absorbing component.

**23.** The body fluid absorbing product according to claim 1, wherein a hydrophilic agent is coated on the surfaces of fibers constituting the top sheet.

**24.** The body fluid absorbing product according to claim 1, wherein the top sheet is water-repellent.

**25.** The body fluid absorbing product according to claim 1, wherein the top sheet has an air permeability of at least 300 $cm^3/(cm^2 \cdot sec)$.

**26.** The body fluid absorbing product according to claim 25, wherein the top sheet has an air permeability of at least 500 $cm^3/(cm^2 \cdot sec)$.

**27.** The body fluid absorbing product according to claim 1, wherein the water absorption rate of the top sheet is 5 seconds or less after 10 industrial washing operations.

**28.** The body fluid absorbing product according to claim 1, further comprising a liquid-impermeable back sheet, the water-absorbing component being disposed between the top sheet and the back sheet.

**29.** The body fluid absorbing product according to claim 28, wherein the back sheet has a limit hydraulic pressure of at least 5 kPa.

**30.** The body fluid absorbing product according to claim 28, wherein the back sheet has a water vapor permeability of at least 4,000 $g/(m^2 \cdot 24\ hours)$.

**31.** The body fluid absorbing product according to claim 28, wherein the back sheet is provided with an antiskid member at the outer face that comes into contact with a cloth.

**32.** The body fluid absorbing product according to claim 28, wherein the top sheet and the water-absorbing component, or the top sheet, the back sheet, and the water-absorbing component or the water-draining layer therebetween are combined by sewing with a water-repellent thread.

**33.** The body fluid absorbing product according to claim 1, wherein the body fluid absorbing product has a structure preventing side leakage.

<div align="center">

**INTERNATIONAL SEARCH REPORT**

</div>

| | International application No. |
|---|---|
| | PCT/JP03/01762 |

**A. CLASSIFICATION OF SUBJECT MATTER**
Int.Cl$^7$  A61F13/53

According to International Patent Classification (IPC) or to both national classification and IPC

**B. FIELDS SEARCHED**

Minimum documentation searched (classification system followed by classification symbols)
Int.Cl$^7$  A61F13/15-13/84

Documentation searched other than minimum documentation to the extent that such documents are included in the fields searched
Jitsuyo Shinan Koho          1926-1996   Toroku Jitsuyo Shinan Koho   1994-2003
Kokai Jitsuyo Shinan Koho   1971-2003   Jitsuyo Shinan Toroku Koho   1996-2003

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y<br>A | JP 2001-286505 A  (Kao Corp.),<br>16 October, 2001 (16.10.01),<br>Full text<br>(Family: none) | 1,2<br>3,4 |
| Y | JP 2-31755 A  (Daio Paper Corp.),<br>01 February, 1990 (01.02.90),<br>Full text<br>(Family: none) | 1,2 |
| Y | JP 1-97255 A  (Toray Industries, Inc.),<br>14 April, 1989 (14.04.89),<br>Full text<br>(Family: none) | 1,2 |

| ☐ Further documents are listed in the continuation of Box C. | ☐ See patent family annex. |
|---|---|

| \* | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 16 May, 2003 (16.05.03) | 27 May, 2003 (27.05.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

EP 1 486 190 A1

## INTERNATIONAL SEARCH REPORT

International application No.

PCT/JP03/01762

**Box I    Observations where certain claims were found unsearchable (Continuation of item 2 of first sheet)**

This international search report has not been established in respect of certain claims under Article 17(2)(a) for the following reasons:

1. ☐ Claims Nos.:

   because they relate to subject matter not required to be searched by this Authority, namely:

2. ☐ Claims Nos.:

   because they relate to parts of the international application that do not comply with the prescribed requirements to such an extent that no meaningful international search can be carried out, specifically:

3. ☐ Claims Nos.:

   because they are dependent claims and are not drafted in accordance with the second and third sentences of Rule 6.4(a).

**Box II    Observations where unity of invention is lacking (Continuation of item 3 of first sheet)**

This International Searching Authority found multiple inventions in this international application, as follows:
  Claims 2, 3, 5-7, 9-25, 27, 28, 33 quote Claim 1.  Therefore, the matter common
to Claims 2, 3, 5-7, 9-25, 27, 28, 33 is the matter given in Claim 1.
  However, a body fluid absorbing product having an air-permeable top sheet
and a water absorbing member disposed therein, which water absorbing member
is composed by laminating a plurality of synthetic fiber non-woven fabric
layers, is disclosed in JP 2001-286505 A (Kao Corporation), 16 October, 2001,
while the use of fiber whose fineness ranges from 0.01 dtex to 2 dtex for
an absorbing member is a known technique in the technical field of absorbing
body products (for example, refer to JP 2-31755 A (Daiou Paper Co., Ltd) 01
(continued to extra sheet)

1. ☐ As all required additional search fees were timely paid by the applicant, this international search report covers all searchable

   claims.

2. ☐ As all searchable claims could be searched without effort justifying an additional fee, this Authority did not invite payment

   of any additional fee.

3. ☒ As only some of the required additional search fees were timely paid by the applicant, this international search report covers

   only those claims for which fees were paid, specifically claims Nos.:  1-4

4. ☐ No required additional search fees were timely paid by the applicant. Consequently, this international search report is

   restricted to the invention first mentioned in the claims; it is covered by claims Nos.:

**Remark on Protest**     ☐     The additional search fees were accompanied by the applicant's protest.

                          ☒     No protest accompanied the payment of additional search fees.

Form PCT/ISA/210 (continuation of first sheet (1)) (July 1998)

13

**INTERNATIONAL SEARCH REPORT**

International application No.

PCT/JP03/01762

Continuation of Box No.II of continuation of first sheet(1)

February, 1990, JP 1-97255 A (Toray Industries Inc.) 14 April, 1989, etc.). AS considered in the light of these facts, as a result, the matter common to Claims 2, 3, 5-7, 9-25, 27, 28, 33 is no better than the prior art. Thus, in the sense of the second sentence in PCT Rule 13. 2 ("special technical feature" means a technical feature such that the individual inventions described in Claims, as a whole, clearly show their contribution to the prior art.), this common matter is not a special technical feature.

Therefore, it is obvious that Claims 2, 3, 5-7, 9-25, 27, 28, 33 do not comply with the requirement of unity of invention.

Form PCT/ISA/210 (extra sheet) (July 1998)